# EUROPEAN PATENT APPLICATION

(11) **EP 2 055 304 A1**
(43) Date of publication of application: **06.05.2009**
(21) Application number: 07806081.1
(22) Date of filing: 24.08.2007
(51) Int. Cl.: A61K 31/497, A61K 9/08, A61K 47/10, A61K 47/12, A61K 47/14, A61K 47/18, A61K 47/20, A61K 47/22

(54) **STABILIZED PHARMACEUTICAL COMPOSITION**

(30) Priority: 25.08.2006 JP 2006229203; 23.08.2007 WO PCT/JP2007/066340
(71) Applicant: Astellas Pharma Inc., Tokyo 103-8411 (JP)
(72) Inventor: MURAI, Makato, Chuo-ku Tokyo, 103-8411 (JP); YONEMOCHI, Yuichi, Chuo-ku Tokyo, 103-8411 (JP); SHAKUSHIRO, Kohsuke, Chuo-ku Tokyo, 103-8411 (JP); KASAI, Akihiro, Chuo-ku Tokyo, 103-8411 (JP)
(74) Representative: Bates, Philip Ian
(86) International application number: PCT/JP2007/066495
(87) International publication number: WO 2008/023807

(57) **Abstract**

[Problem]

A stable liquid-form pharmaceutical composition wherein the decomposition with time is prevented and a stabilization method thereof is provided, for providing a liquid-form pharmaceutical composition of 1-(2-methoxyethyl)-2-methyl-4,9-dioxo-3-(pyrazin-2-ylmethyl)-4,9-dihydro-1H-naphtho[2,3-d]imidazol-3-ium or a pharmaceutically acceptable salt thereof to a clinical fields.

[Means for Solution]

Related are a stable pharmaceutical composition of 1-(2-methoxyethyl)-2-methyl-4,9-dioxo-3-(pyrazin-2-ylmethyl)-4,9-dihydro-1H-naphtho[2,3-d]imidazol-3-ium or a pharmaceutically acceptable salt thereof, containing one kind or two or more kinds selected from the group consisting of carbazochrome sodium sulfonate and derivatives thereof, vitamin C's, butylhydroxylanisol, vitamin E's, Vitamin P's, gallic acid, propyl gallate, alpha-thioglycerine and cysteine hydrochloride, as well as a lyophilized product thereof and a stabilizing method thereof.

## Description

### Technical Field

The present invention relates to a stable pharmaceutical composition of 1-(2-methoxyethyl)-2-methyl-4,9-dioxo-3-(pyrazin-2-ylmethyl)-4,9-dihydro-1H-naphtho[2,3-d]imidazol-3-ium (hereinafter, referred to as compound A) or a pharmaceutically acceptable salt thereof, which is characterized by containing one kind or two or more kinds selected from the group consisting of carbazochrome sodium sulfonate and derivatives thereof, vitamin C's, butylhydroxylanisol, vitamin E's, Vitamin P's, gallic acid, propyl gallate, alpha-thioglycerine and cysteine hydrochloride; a lyophilized product thereof; and a stabilizing method thereof.

### Background Art

1-(2-methoxyethyl)-2-methyl-4,9-dioxo-3-(pyrazin-2-ylmethyl)-4,9-dihydro-1H-naphtho[2,3-d]imidazol-3-ium chloride (chloride of compound A) is a compound reported as an antitumor agent because it has excellent cancer proliferation-inhibitory activity in vivo, as well as it is low in toxicity (see Patent Document 1).
The chloride of the compound A has hygroscopicity and is unstable to humidity such as increase of decomposed substances in long-term storage, whereas a bromide of the compound A exhibits no hygroscopicity and excellent storage stability and thus has been reported to be useful as a bulk material for producing pharmaceuticals (See Patent Document 2).

However, it is neither disclosed nor even suggested in Patent Documents 1 and 2 that a liquid-form pharmaceutical composition of the compound A is unstable to light and thus significantly decomposes with time.
Patent Document 1: Pamphlet of International Publication No. 01/60803
Patent Document 2: Pamphlet of International Publication No. 04/92160

### Disclosure of the Invention

### Problem that the Invention is to Solve

In order to provide a liquid-form pharmaceutical composition of the compound A or a pharmaceutically acceptable salt thereof to clinical fields, it has been necessary to provide a stable liquid-form pharmaceutical composition in which the decomposition with time is prevented.
Specifically, it has been desired that a preparation is stable even in case of long-term, for example 24 hours, administration after it is opened or prepared by dissolving a lyophilized product. More specifically, it has been necessary to provide a liquid-form pharmaceutical composition in which the residual ratio of the compound A is 80% or more, more preferably 90% or more, particularly preferably 95% or more, in a state where the preparation is not shielded from light for long time after being opened or prepared by dissolving a lyophilized product.

### Means for Solving the Problem

In order to exhibit the efficacy of the compound A, it is necessary to continually carry out an intravenous injection for a long period of time. By using such a special administration method, it was found that a liquid-form pharmaceutical composition of the compound A is vulnerable to light.
In case of administering a drug that readily decomposes by light, a general way to prevent the decomposition is to utilize the packaging to block light, such as the use of a light-shielding cover or placing of the preparation into a brown glass bottle. However, these methods are inconvenient in handling, and also there is a risk of drug decomposition if the light shielding is deficient. In order to fundamentally solve these problems, it is necessary to produce a preparation having improved light stability by devising the preparation during the process of formulating the compound A.
In case of a solid pharmaceutical composition, methods such as coating with iron oxide or titanium oxide have been widely employed in order to improve light stability, but these methods cannot be employed for liquid-form pharmaceutical compositions. In addition, there are no additives which can serve as a universal light stabilizer and widely used for all drugs. Therefore, there is no general methodology for stabilizing compounds of various structures and unstable to light in a solution.
Thus, most of the injection drugs that are unstable to light are sold without applying any improvement to the pharmaceutical formulation, and the current situation is light-shielding by physical methods during storage and during administration, for example, as in the cases of anti-tumor agent (trade name: Dacarbadine for Intravenous Injection, produced by Kyowa Hakko Kogyo Co., Ltd.) or vitamin preparations (trade name: Kaytwo N for Intravenous Injection, produced by Eisai; and Vitaject, produced by Terumo).
As an exceptional example where stabilization has been achieved, although very few are reported, there are methods of adding other compounds to the drug as a stabilizer. For example, there are injection drugs in which nitroprusside or sitafloxacin is stabilized against light, and the like (for example, see JP-A-7-126017, W001080858 and the like).
However, the chemical structures of these drugs and the kinds of the light stabilizer are respectively different from each other, and thus it is believed that the stabilization mechanisms are also different. Therefore, it is not considered that such methods provide light stabilization in any drug. Furthermore, a similarity in chemical structure between those two drugs and the compound A is not found.
From these backgrounds, it was expected that it is difficult to achieve an improvement in the light stability of the compound A by means of adding a light stabilizer to the preparation.
After extensive investigation regarding the photodegradation reaction of the compound A, the present inventors have discovered that, among various compounds added, particular substances, i.e., quercetin, ascorbic acid, gallic acid, carbazochrome sodium sulfonate, butylhydroxylanisol, 6-hydroxy-2,5,7,8-tetramethylchromane-2-carbonic acid, alpha-thioglycerine and cysteine hydrochloride have light stabilizing effect.
Furthermore, among those substances mentioned above, further investigation on the stabilization mechanism was made regarding quercetin, ascorbic acid, gallic acid, carbazochrome sodium sulfonate, butylhydroxylanisol, and 6-hydroxy-2,5,7,8-tetramethylchromane-2-carbonic acid. As a result, a possibility of correlation between light stabilizing effect and radical-scavenging ability was suggested in quercetin, ascorbic acid, gallic acid, butylhydroxylanisol, and 6-hydroxy-2,5,7,8-tetramethylchromane-2-carbonic acid.
Meanwhile, although the correlation between the light stabilizing effect and the radical-scavenging ability was low in carbazochrome sodium sulfonate, it was discovered surprisingly that it has a strong light stabilizing effect for the compound A. From this, although the mechanism of the light stabilizing effect of carbazochrome sodium sulfonate is yet unclear, it was found to be an excellent substance for improving the light stability of the compound A.

In other words, the present invention relates to:
1. A stable pharmaceutical composition of 1-(2-methoxyethyl)-2-methyl-4,9-dioxo-3-(pyrazin-2-ylmethyl)-4,9-dihydro-1H-naphtho[2,3-d]imidazol-3-ium or a pharmaceutically acceptable salt thereof, which is characterized by containing one kind or two or more kinds selected from the group consisting of carbazochrome sodium sulfonate and derivatives thereof, vitamin C's, butylhydroxylanisol, vitamin E's, vitamin P's, gallic acid, propyl gallate, alpha-thioglycerine and cysteine hydrochloride,
2. The pharmaceutical composition according to claim 1, which is characterized by containing one kind or two or more kinds selected from the group consisting of carbazochrome sodium sulfonate and derivatives thereof, vitamin C's, butylhydroxylanisol, vitamin E's, vitamin P's, gallic acid and propyl gallate,
3. The pharmaceutical composition according to claim 1, which is characterized by containing one kind or two or more kinds selected from the group consisting of carbazochrome sodium sulfonate and derivatives thereof, vitamin C's and butylhydroxylanisol,
4. The pharmaceutical composition according to claim 1, which is characterized by containing carbazochrome sodium sulfonate and derivatives thereof,
5. The pharmaceutical composition according to Claims 1 to 4, wherein the mixing amount of 1-(2-methoxyethyl)-2-methyl-4,9-dioxo-3-(pyrazin-2-ylmethyl)-4,9-dihydro-1H-naphtho[2,3-d]imidazol-3-ium or a pharmaceutically acceptable salt thereof, is 0.01 mg/ml or more,
6. The pharmaceutical composition according to claim 4 or 5, wherein the mixing amount of carbazochrome sodium sulfonate and derivatives thereof is 0.01 parts by weight or more based on 1-(2-methoxyethyl)-2-methyl-4,9-dioxo-3-(pyrazin-2-ylmethyl)-4,9-dihydro-1H-naphtho[2,3-d]imidazol-3-ium or a pharmaceutically acceptable salt thereof,
7. A pharmaceutical composition which can be obtained by lyophilizing the pharmaceutical composition according to claims 1 to 6,
8. The pharmaceutical composition according to claims 1 to 7, which is for intravenous administration,
9. The pharmaceutical composition according to claim 8, which is injected for 4 hours or more,
10. The pharmaceutical composition according to claims 1 to 7, which is packaged for intravenous administration of 4 hours or more,
11. A method of stabilizing a pharmaceutical composition containing 1-(2-methoxyethyl)-2-methyl-4,9-dioxo-3-(pyrazin-2-ylmethyl)-4,9-dihydro-1H-naphtho[2,3-d]imidazol-3-ium or a pharmaceutically acceptable salt thereof, which is characterized by mixing one kind or two or more kinds selected from the group consisting of carbazochrome sodium sulfonate and derivatives thereof, vitamin C's, butylhydroxylanisol, vitamin E's, vitamin P's, gallic acid, propyl gallate, alpha-thioglycerine and cysteine hydrochloride.

Hereinafter, the composition of the present invention will be described in detail.
Since the present invention is a liquid-form pharmaceutical composition, the pharmaceutically acceptable salt of the compound A according to the present invention is not particularly limited, and bromide which exhibits no hygroscopicity and excellent long-term storage stability is preferable as the bulk material for production.
There are no particular limitations on the liquid-form pharmaceutical composition of the present invention, as long as it is a liquid-form pharmaceutical composition. More specifically, in addition to those for systemic administration such as a pharmaceutical composition for injection which includes drip infusion and the like or a liquid-form pharmaceutical composition for oral administration, those useful for topical administration such as a liquid agent for external use or sprays can be exemplified. A preferred form of the pharmaceutical composition of the present invention is a pharmaceutical composition for injection, and more specifically, a pharmaceutical composition for injection which is useful for intramuscular or intravenous administration. Furthermore, the pharmaceutical composition of the present invention includes lyophilized products which can be dissolved suitably in physiological saline or the like before administration so as to be prepared into an adequate concentration described later.

The mixing amount of the compound A for the liquid-form pharmaceutical composition of the present invention is not particularly limited, considering the therapeutic method as the antitumor agent in clinical trials made to the present. An aqueous solution of the compound A of 0.01 mg/ml or more may be mentioned. The concentration of the aqueous solution of the compound A is preferably 0.01 mg/ml to 100 mg/ml, more preferably 0.01 mg/ml to 5 mg/ml, particularly preferably 0.05 mg/ml to 1 mg/ml, and most preferably 0.05 mg/ml to 0.5 mg/ml.
The administration method of the liquid-form pharmaceutical composition of the present invention is not particularly limited. A continuous intravenous administration by drip infusion or the like for 7 days may be considered. The long-term administration is not preferable when considering the stability of the compound A after opening a liquid-form pharmaceutical composition or after dissolving a lyophilized product for preparation, but it is preferable to carry out administration as long as possible without replacing a drug solution when considering the labor by nurses or pharmacists in medical institutions or the prevention of medical accidents. Therefore, more specifically, the pharmaceutical composition of the present invention may be administered intravenously by drip infusion or the like each time over 4 hours or more, preferably 10 hours or more, more preferably 15 hours or more, and particularly preferably 24 hours or more.
In other words, it can be said that the pharmaceutical composition of the present invention is a composition which is stable during the above-mentioned term after it being opened or prepared by dissolving a lyophilized product.

As for the light stabilizer of the compound A which to be included in the liquid-form pharmaceutical composition of the present invention, there are vitamin C's, butylhydroxylanisol, vitamin E's, vitamin P's, gallic acid, propyl gallate, alpha-thioglycerine, cysteine hydrochloride and derivatives thereof. Preferred are vitamin C's, butylhydroxylanisol, vitamin E's, vitamin P's, gallic acid, propyl gallate and derivatives thereof, and more preferred are vitamin C's, butylhydroxylanisol and derivatives thereof. The stabilizers mentioned above can be suitably used by mixing one kind or two or more kinds thereof.
Vitamin C's mentioned herein are ascorbic acid palmitate, ascorbyl stearate, erythoric acid and the like; vitamin E's are 6-hydroxy-2,5,7,8-tetramethylchromane-2-carbonic acid (trade name Trolox), dl-α-tocopherol, d-δ-tocopherol, tocorol, tocotrienol and the like; and vitamin P's are quercetin, rutin and flavonoids.

The mixing amount of the light stabilizer for the above-mentioned compound A in the pharmaceutical composition of the present invention is not particularly limited as long as it is in a range that can be dissolved in water or in water added with a solubilizer such as ethanol and exhibit stabilization effect on the compound A. Specifically, the weight ratio of the stabilizer for the compound A is 1.3 to 13 parts by weight for alpha-thioglycerine, 1 to 10 parts by weight for cysteine hydrochloride, 0.02 to 20 parts by weight and preferably 0.2 to 20 parts by weight for vitamin C's, 0.25 to 2.5 parts by weight for vitamin E's, 0.0004 to 0.4 parts by weight and preferably 0.04 to 0.4 parts by weight for vitamin P's, 0.04 to 4 parts by weight and preferably 0.4 to 4 parts by weight for butylhydroxyanisol, 0.005 to 5 parts by weight and preferably 0.05 to 5 parts by weight for gallic acid or propyl gallate.

In addition, as the light stabilizer of the compound A included in the liquid-form pharmaceutical composition of the present invention, carbazochrome sodium sulfonate and derivatives thereof may be exemplified. As for derivatives of carbazochrome sodium sulfonate, there are carbazochrome, adrenochrome monoaminoguanidine methanesulfonate and the like. Since carbazochrome sodium sulfonate shows low reactivity with the compound A and does not show change in formulation, it is especially stable under the condition of long-term storage, and is most preferable as the stabilizer of the present invention.
The mixing amount of carbazochrome sodium sulfonate and derivatives thereof in the pharmaceutical composition of the present invention is, based on the compound A, 0.01 parts by weight or more and, when considering the stabilization effect after dilution to a clinical injection concentration, it is preferably 0.05 parts by weight or more, particularly preferably in the range of 0.1 to 1.0 parts by weight, and most preferably in the range of 0.2 to 0.5 parts by weight or more. Furthermore, when considering the previous examples of usage, it is desirably mixed by 20 parts by weight or less, preferably in the range of 0.5 parts by weight or less. The stabilizer mentioned above can be suitably used by mixing one kind or two or more kinds thereof.

The production method of the liquid-form pharmaceutical composition which contains an aqueous solution of the compound A of the present invention will be described below.
An acidic buffering agent or an acidic pH regulator, a tonicity agent and one or more light stabilizers mentioned in claims of the present invention are dissolved in an injection solvent so that each final concentration in the drug solution becomes the desired concentration, and then pH is adjusted by adding an alkaline pH regulator to produce an acidic buffer solution for drug solution preparation. Therein, the raw material for producing the compound A is dissolved, and the injection solvent is added adequately so that the whole amount is adjusted to become a predetermined amount. This drug solution is filled into a sealed container and then subjected to a suitable sterilization, or subjected to a suitable sterile manipulation and then filled into a sealed container, to give a liquid-form pharmaceutical composition of the compound A.
For the procedure described above, as the acidic buffering agent or acidic pH regulator, lactic acid, citric acid, phosphoric acid, tartaric acid, diluted hydrochloric acid or the like can be used. As the tonicity agent, sodium chloride, magnesium chloride, sodium bromide, fructose, lactose, glucose, D-sorbitol, nicotinamide or the like may be used. In addition, the alkaline pH regulator is not particularly limited as long as it is an alkaline substance used as a pH regulator for medical drugs, such as sodium hydroxide, magnesium hydroxide, potassium hydroxide and the like.
The order of adding compound A and the stabilizer of the present invention are not limited to this order. In addition, suitable solubilization method may be employed when adding the light stabilizer mentioned in the claims of the present invention by necessary amounts. Examples of the solubilizer include ethanol, propyleneglycol, conc. glycerine, polyethyleneglycol, dimethylacetamide, polysorbate, polyoxyethylene-hardened castor oil, cyclodextrin and the like.

In the case of using the liquid-form pharmaceutical composition of the present invention for injection, additives such as soothing agents like creatinine, benzylalcohol, mepivacaine hydrochloride, xylocaine hydrochloride, lidocaine and the like, or preserving agents like benzylalcohol, methylparabenzoate, propylparabenzoate, gentisic acid ethanolamide, thimerosal, chlorobutanol and the like may be added as necessary. In addition, additives of hydrophilic low molecules or the like, such as glucose, sodium chloride, glycine, mannitol and the like may be added as necessary to alleviate the local toxicity (for example, see JP-A-11-193233).

Furthermore, the liquid-form pharmaceutical composition of the present invention may be added with physiologically acceptable excipients to give a lyophilized product. The excipient is not particularly limited as long as it typically enhances the moldability of the lyophilized substance, and examples thereof may include saccharides such as mannitol, inositol, maltose, sucrose, lactose, cyclodextrin, dextran 40 and the like, and amino acids such as glycine, alanine, valine, methionine and the like. These lyophilized preparations are prepared by being suitably dissolved in physiological saline or the like before administering into a patient.
Furthermore, the pharmaceutical composition of the present invention shows excellent miscibility with electrolyte fluids such as physiological saline, glucose fluid and the like, saccharide fluids and other fluids, and thus it is possible to use it by mixing with these fluids.

### Brief Description of the Drawings

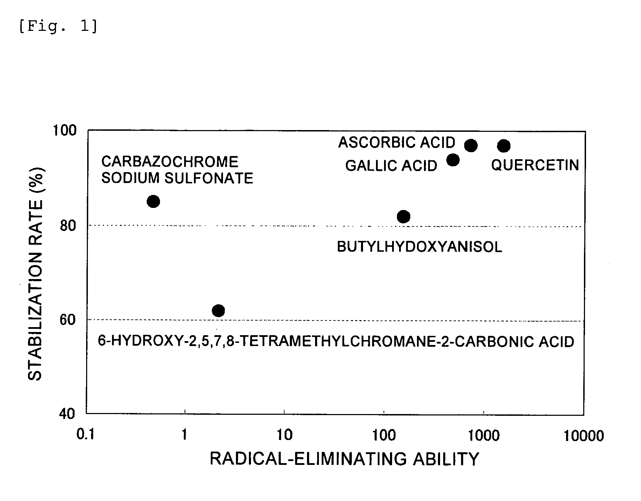

[Fig. 1] Fig. 1 shows a relationship between various stabilizers and radical-scavenging ability.

### Best Mode for Carrying Out the Invention

The liquid-form pharmaceutical composition of compound A or a pharmaceutically acceptable salt thereof according to the present invention will be described in detail. Hereinafter, the present invention will be described in further detail with reference to Examples and Comparative Examples, but the present invention is not intended to be limited by these.

### [Comparative Example 1]

After dissolving lactic acid and sodium chloride in water so that the final concentration in the drug solution each becomes 9 and 3.5 mg/mL, pH was regulated to 3.6 by adequately adding 1 mol/L of sodium hydroxide solution to produce a lactic acid buffer solution for drug solution preparation. A bromide of compound A was dissolved in this, and injection solvent was adequately added so that the final concentration becomes 10 mg/mL to prepare the drug solution of compound A. This was filled into a glass vial, spigot-seamed and then sterilized with high-pressured steam to obtain the pharmaceutical composition of compound A.

### [Comparative Example 2]

After dissolving lactic acid and sodium chloride in water so that the final concentration in the drug solution each becomes 9 and 3.5 mg/mL, pH was regulated to 3.6 by adequately adding 1 mol/L of sodium hydroxide solution to prepare a lactic acid buffer solution of pH 3.6. Additionally, 3mL of this solution was mixed with 3 mL of the pharmaceutical composition of the Comparative Example 1 mentioned above (compound A, 10 mg/mL), added to a white glass container and spigot-seamed to obtain a comparative pharmaceutical composition of Comparative Example 2.

### [Comparative Example 3]

D-fructose, citric acid or menadione sodium bisulfite was dissolved in a 9 mg/mL lactic acid solution, and pH was regulated to 3.6 by adequately adding a sodium hydroxide solution. Additionally, 3 mL of the aforementioned stabilizer was mixed with 3 mL of the comparative pharmaceutical composition of the Comparative Example 1 mentioned above (compound A, 10 mg/mL), added to a white glass container and spigot-seamed to obtain the pharmaceutical composition of Comparative Example 3.

### Example 1

Quercetin was dissolved in ethanol. Additionally, 3 mL of the aforementioned stabilizer was mixed with 3 mL of the composition of the Comparative Example 1 mentioned above (compound A, 10 mg/mL), added to a white glass container and spigot-seamed to obtain the pharmaceutical composition of Example 1.

### Example 2

Ascorbic acid was dissolved in a 9 mg/mL lactic acid solution, and pH was regulated to 3.6 by adequately adding a sodium hydroxide solution. Additionally, 3 mL of the aforementioned stabilizer was mixed with 3 mL of the pharmaceutical composition of the Comparative Example 1 mentioned above (compound A, 10 mg/mL), added to a white glass container and spigot-seamed to obtain the pharmaceutical composition of Example 2.

### Example 3

After gallic acid was dissolved in ethanol, and then dissolved in a lactic acid buffer solution of which the pH was regulated to 3.6 in advance by adequately adding a sodium hydroxide solution to 9 mg/mL of lactic acid solution. Additionally, 3 mL of the aforementioned stabilizer was mixed with 3 mL of the comparative pharmaceutical composition of the Comparative Example 1 mentioned above (compound A, 10 mg/mL), added to a white glass container and spigot-seamed to obtain the pharmaceutical composition of Example 3.

### Example 4

Aside from changing ascorbic acid of Example 2 to carbazochrome sodium sulfonate, the same method as in Example 2 was used to obtain the pharmaceutical composition of Example 4.

### Example 5

Aside from changing quercetin of Example 1 to butylhydroxyanisol, the same method as in Example 1 was used to obtain the pharmaceutical composition of Example 5.

### Example 6

Aside from changing quercetin of Example 1 to 6-hydroxy-2,5,7,8-tetramethylchromane-2-carbonic acid, the same method as in Example 1 was used to obtain the pharmaceutical composition of Example 6.

### Example 7

Aside from changing ascorbic acid of Example 2 to cysteine hydrochloride, the same method as in Example 2 was used to obtain the pharmaceutical composition of Example 7.

### Example 8

Aside from changing ascorbic acid of Example 2 to alpha-thioglycerine, the same method as in Example 2 was used to obtain the pharmaceutical composition of Example 8.

### «Effects of Various Stabilizers on light stability of Compound A Solution»

### (Experimental Example 1)

Each sample was stored upside-down under a condition of D65 lamp/1000 lux in a constantly changing room temperature for 24 hours to evaluate the stability by measuring the increased amounts of photolyzed substances. The measurement of photolyzed substances was carried out using HPLC.

### (HPLC Conditions)

Mobile phase (liquid A) : 0.1% trifluoroacetic acid aqueous solution/acetonitrile=9:2, (liquid B) acetonitirle, gradient% (capacity) B : 0% (0 to 60 min)/linear 75% (60 to 80 min)/linear 0% (80 to 100 min), column : TSK-gel ODS-80TsQA, 4.6x150 mm, particle diameter 5 µm (produced by Tosoh Corp.), flow rate : 1 mL/min, column temperature : 40°C, detection : UV 270 nm.

### (Result of Experimental Example 1)

The stabilizers used in Comparative Example 2 and Examples 1 to 8, weight ratio (parts by weight) of the stabilizers for compound A and photolyzed substance amount are shown in Table 1.

**[Table 1]**

| | Stabilizer | Additives/ Compound A | Photolyzed Substance Amount (%) | Stabilization Rate (%) * |
|---|---|---|---|---|
| Comparative Example 2 | Not-Added | - | 3.4 | |
| Comparative Example 3 | D-fructose | 20.46 | 2.9 | 15 |
| | Citric Acid | 23.86 | 6.2 | <0 |
| | Menadione Sodium Bisulfite | 0.75 | 3.9 | <0 |
| Example 1 | Quercetin | 0.38 | Less than 0.1 | 97 or more |
| Example 2 | L-Ascorbic Acid | 20.00 | 0.1 | 97 |
| Example 3 | Gallic Acid | 4.27 | 0.2 | 94 |
| Example 4 | Carbazochrome Sodium Sulfonate | 0.43 | 0.5 | 85 |
| Example 5 | Butylhydoxyanisol (BHA) | 4.09 | 0.6 | 82 |
| Example 6 | 6-Hydroxy-2,5,7,8-Tetramethylchromane-2-Carbonic Acid | 2.50 | 1.3 | 62 |
| Example 7 | L-Cysteine Hydrochloride | 9.97 | 1.3 | 62 |
| Example 8 | Alpha-Thioglycerine | 12.28 | 1.5 | 56 |

| | | | | |
|---|---|---|---|---|
| * Stabilization Rate (%)=100-(photolyzed substance amount/photolyzed substance amount of the non-added material×100) | | | | |

Predetermined amount of stabilizer was added to compound A drug solution (5 mg/mL), and after irradiation by light of D65 lamp/1000 lux for 24 hours, the photolyzed substance amount was measured, in the non-added case under the conditions of this test, 3.4% of photolyzed substances were generated. Among the added light stabilizers, some compounds showed no light stabilizing effect, but when quercetin, ascorbic acid, gallic acid, carbazochrome sodium sulfonate, butylhydroxyanisol and 6-hydroxy-2,5,7,8-tetramethylchromane-2-carbonic acid were added for Example, the generation of photolyzed substances were suppressed, and thus the light stabilizing effect was exhibited.
Furthermore, when cysteine hydrochloride or alpha-thioglycerine were added, generation of photolyzed substances were suppressed and exhibited light stabilizing effect, but a tendency of increasingly giving substances other than photolyzed substances were shown.
As a result of the present test, it was suggested that in the combination of the compound A and a specific substance, light stabilization could be achieved.

### (Experimental Example 2)

### «Radical-Scavenging Ability Confirmation Test by Light Absorbance Measurement Using DPPH»

A radical-scavenging ability test using DPPH (1,1-diphenyl-2-picrylhydrazyl) was performed on stabilizers exhibiting light stabilizing effects. DPPH is a kind of nitrogen radical, and is used as a free radical reagent (see TAKEUCHI Tokuo et al., "Studies on the DPPH Radical Scavenging ability of Miso", Bulletin of Gifu Women's College, 33, pp. 115-122(2004)).
0.5 mL of test solution prepared by adding the stabilizer of the present invention for an adequate concentration and 0.5 mL of 0.8 mmol/L DPPH solution was diluted to 2 mL so that ethanol:water ratio becomes 1:1 and mixed, and the light absorbance of 520 nm was measured after 30 minutes. The amount of stabilizer (mol) needed to fade the light absorbance by 50% was calculated, this value was subtracted by the amount of stabilizer (mol) of the compound A solution during the light stability test, and the resulting value was set as the radical-scavenging ability.

### (Result of Experimental Example 2)

A plot result regarding each sample solution, where the vertical axis is the stabilization rate during the light stability test and the horizontal axis is the radical scavenging ability, is shown in Fig. 1. Regarding quercetin, ascorbic acid, gallic acid, butylhydroxyanisol and 6-hydroxy-2,5,7,8-tetramethylchromane-2-carbonic acid, a possibility of correlation between light stabilizing effect and radical scavenging ability was indicated.
On the other hand, regarding carbazochrome sodium sulfonate, no correlation between light stabilizing effect and radical scavenging ability was found. Judging from this, it seems that carbazochrome sodium sulfonate has a certain function that brings about a light stabilizing effect aside from the radical-scavenging ability.

### [Comparative Example 4]

3 mL of physiological saline was mixed with 3 mL of the pharmaceutical composition of the Comparative Example 1 mentioned above (compound A, 10 mg/mL), added to a white glass container and spigot-seamed to obtain the comparative pharmaceutical composition of Comparative Example 4.

### Example 9

3 mL of the pharmaceutical composition of the Comparative Example 1 (compound A, 10 mg/mL) was added with 3 mL of physiological saline containing a predetermined amount of carbazochrome sodium sulfonate, added to a white glass container and spigot-seamed to obtain the pharmaceutical composition of Example 9.

### (Experimental Example 3)

Among the stabilizers of the present invention with high light stabilizing effects, a further examination was carried out on carbazochrome sodium sulfonate, which was considered to have excellent stability, and in particular, low reactivity (no mixing variation) with compound A.
An examination was carried out concerning the additive amount of carbazochrome sodium sulfonate needed to obtain a light stabilizing effect.
The samples of Comparative Example 4 and Example 9 were stored upside-down under a condition of white fluorescent lamp/1000 lux in a constantly changing room temperature for 168 hours to evaluate the stability by measuring the residual ratio of compound A. The quantitative determination of compound A in each solution was carried out using HPLC.

### (HPLC Conditions)

Mobile phase: 0.1% trifluoroacetic acid aqueous solution/acetonitrile=9:2, column: TSK-gel ODS-80TsQA, 4.6x150 mm, particle diameter 5 µm (produced by Tosoh Corp.), flow rate: 1 mL/min, column temperature: 40°C, detection: UV 270 nm.

### (Result of Experimental Example 3)

The results are shown in Table 2.
Residual Ratio of Compound A (%) (Concentration of Compound A: 5 mg/mL Diluted in Physiological Saline)

**[Table 2]**

| Carbazochrome sodium sulfonate /Compound A | Residual Ratio After 168 Hours of Irradiation | Standard Deviation | Student's T-Test (n=3) |
|---|---|---|---|
| Comparative Example 4 (0) | 86.7 | 0.03 | P<0.001 |
| Example 9 (0.01) | 88.5 | 0.08 | |

When the weight ratio between carbazochrome sodium sulfonate and compound A was 0.01, photodegradation of the compound A was significantly suppressed. Judging from this, it was found that in order to obtain a light stabilizing effect, at least 0.01 parts by weight of carbazochrome sodium sulfonate is required regarding the weight ratio with compound A.

### Example 10

After the final concentration of the pharmaceutical composition of compound A of Comparative Example 1 was diluted to become 0.1 mg/mL with a physiological saline added with a predetermined amount of carbazochrome sodium sulfonate of Table 3, it was added to a white glass container and spigot-seamed to obtain the pharmaceutical composition of Example 10.

### Example 11

After the final concentration of the pharmaceutical composition of compound A of Comparative Example 1 was diluted to become 0.1 mg/mL with a physiological saline added with a predetermined amount of carbazochrome sodium sulfonate of Table 3, it was added to a white glass container and spigot-seamed to obtain the pharmaceutical composition of Example 11.

### Example 12

After the final concentration of the pharmaceutical composition of compound A of Comparative Example 1 was diluted to become 0.1 mg/mL with a physiological saline added with a predetermined amount of carbazochrome sodium sulfonate of Table 3, it was added to a white glass container and spigot-seamed to obtain the pharmaceutical composition of Example 12.

### [Comparative Example 5]

After the final concentration of the pharmaceutical composition of compound A of Comparative Example 1 was diluted to become 0.1 mg/mL with a physiological saline, it was added to a white glass container and spigot-seamed to obtain the comparative pharmaceutical composition of Comparative Example 5.

### (Experimental Example 4)

A confirmation of whether a pharmaceutical composition containing carbazochrome sodium sulfonate as a stabilizer of the present invention is stable against light even when diluted during administration was carried out.
The samples of Comparative Example 4 and Examples 10 to 12 were stored upside-down under a condition of white fluorescent lamp/1000 lux at the room temperature and leaving the temperature to change for 168 hours to evaluate the stability by measuring the residual ratio of compound A. The quantitative determination of the sample solution was carried out using HPLC.

### (HPLC Conditions)

Mobile phase (liquid A) : 0.1% trifluoroacetic acid aqueous solution/acetonitrile=9:2, (liquid B) acetonitirle, gradient% (capacity) B : 0% (0 to 60 min)/linear 75% (60 to 80 min)/linear 0% (80 to 100 min), column : TSK-gel ODS-80TsQA, 4.6x150 mm, particle diameter 5 µm (produced by Tosoh Corp.), flow rate : 1 mL/min, column temperature : 40°C, detection : UV 270 nm.

### (Result of Experimental Example 4)

The results are shown in Table 3. Residual Ratio of Compound A (%) (Concentration of Compound A : 0.1 mg/mL Diluted in Physiological Saline)

**[Table 3]**

| Carbazochrome sodium sulfonate /Compound A | Light Irradiation Time and Residual Ratio (%) | | | |
|---|---|---|---|---|
| | 0 Hours | 4 Hours | 15 Hours | 24 Hours |
| Comparative Example 5 (0) | 100 | 98.0 | 92.2 | 88.3 |
| Example 10 (0.05) | 100 | 98.4 | 95.1 | 92.9 |
| Example 11 (0.5) | 100 | 99.7 | 98.8 | 98.1 |
| Example 12 (25) | 100 | 99.9 | 99.7 | 99.7 |

In the case of Comparative Example 5 where compound A is diluted to a clinically assumed administration concentration (0.1 mg/ml), it was found that decomposition progressed when the light irradiation time was lengthened. When carbazochrome sodium sulfonate was added, light stability was enhanced with the increase of additive amounts.
In the case of Comparative Example 5 where carbazochrome sodium sulfonate was not added, the residual ratio dropped to 92% after 15 hours of light irradiation, and moreover, the residual ratio dropped to 88% after 24 hours.
On the other hand, in the case of Example 10 where the weight ratio between carbazochrome sodium sulfonate and compound A was 0.05, the residual ratio was 95% after 15 hours, and the residual ratio was 93% after 24 hours; light stability in both were enhanced when compared to a non-added case.
In the case of Example 11, in which the concentration was even further increased, where the weight ratio between carbazochrome sodium sulfonate and compound A was 0.5, the residual ratio was 99% after 15 hours and 98% after 24 hours.
In the case of Example 12, in which the concentration was even further increased, where the weight ratio between carbazochrome sodium sulfonate and compound A was 25, the residual ratio showed almost no changes after 15 hours and 24 hours.
It can be thought that, according to the use of the liquid-form pharmaceutical composition containing carbazochrome sodium sulfonate, the light stability of a drug solution at the time of administration can be enhanced, and the duration of quality maintenance can be increased.

### Industrial Applicability

A technical aspect of the present invention is to stabilize a composition itself by mixing a certain compound with the liquid-form pharmaceutical compositions of 1-(2-methoxyethyl)-2-methyl-4,9-dioxo-3-(pyrazin-2-ylmethyl)-4,9-dihydro-1H-naphtho[2,3-d]imidazol-3-ium or a pharmaceutically acceptable salt thereof. Since it became possible to provide a liquid-form pharmaceutical composition which is stable over time, particularly in cases of long-term continuous administration after opening the preparation, it provides a significant industrial effect.

## Claims

1. A stable pharmaceutical composition of 1-(2-methoxyethyl)-2-methyl-4,9-dioxo-3-(pyrazin-2-ylmethyl)-4,9-dihydro-1H-naphtho[2,3-d]imidazol-3-ium or a pharmaceutically acceptable salt thereof, comprising:
one kind or two or more kinds selected from the group consisting of carbazochrome sodium sulfonate and derivatives thereof, vitamin C's, butylhydroxylanisol, vitamin E's, vitamin P's, gallic acid, propyl gallate, alpha-thioglycerine and cysteine hydrochloride.

2. The pharmaceutical composition according to Claim 1, comprising:
one kind or two or more kinds selected from the group consisting of carbazochrome sodium sulfonate and derivatives thereof, vitamin C's, butylhydroxylanisol, vitamin E's, vitamin P's, gallic acid and propyl gallate.

3. The pharmaceutical composition according to Claim 1, comprising:
one kind or two or more kinds selected from the group consisting of carbazochrome sodium sulfonate and derivatives thereof, vitamin C's and butylhydroxylanisol.

4. The pharmaceutical composition according to Claim 1, comprising carbazochrome sodium sulfonate and derivatives thereof.

5. The pharmaceutical composition according to Claims 1 to 4, wherein the mixing amount of 1-(2-methoxyethyl)-2-methyl-4,9-dioxo-3-(pyrazin-2-ylmethyl)-4,9-dihydro-1H-naphtho[2,3-d]imidazol-3-ium or a pharmaceutically acceptable salt thereof, is 0.01 mg/ml or more.

6. The pharmaceutical composition according to Claim 4 or 5, wherein the mixing amount of carbazochrome sodium sulfonate and derivatives thereof is 0.01 parts by weight or more based on 1-(2-methoxyethyl)-2-methyl-4,9-dioxo-3-(pyrazin-2-ylmethyl)-4,9-dihydro-1H-naphtho[2,3-d]imidazol-3-ium or a pharmaceutically acceptable salt.

7. A pharmaceutical composition which is obtainable by lyophilization of the pharmaceutical composition according to Claims 1 to 6.

8. The pharmaceutical composition according to Claims 1 to 7, which is for intravenous administration.

9. The pharmaceutical composition according to Claim 8, which is administered for 4 hours or more.

10. The pharmaceutical composition according to Claims 1 to 7, which is packaged for intravenous administration of 4 hours or more.

11. A method of stabilizing a pharmaceutical composition containing 1-(2-methoxyethyl)-2-methyl-4,9-dioxo-3-(pyrazin-2-ylmethyl)-4,9-dihydro-1H-naphtho[2,3-d]imidazol-3-ium or a pharmaceutically acceptable salt thereof, which is **characterized by** mixing one kind or two or more kinds selected from the group consisting of carbazochrome sodium sulfonate and derivatives thereof, vitamin C's, butylhydroxylanisol, vitamin E's, Vitamin P's, gallic acid, propyl gallate, alpha-thioglycerine and cysteine hydrochloride.
